# DEMANDE DE BREVET EUROPEEN

(11) **EP 3 913 062 A1**
(43) Date de publication de la demande: **24.11.2021**
(21) Numéro de dépôt: 21173327.4
(22) Date de dépôt: 11.05.2021
(51) Int. Cl.: C12P 5/02, C02F 11/04

(54) **RECUPERATION DE METHANE DE DIGESTATS SOLIDES**

(30) Priorité: 20.05.2020 FR 2005137
(71) Demandeur: L'AIR LIQUIDE, SOCIETE ANONYME POUR L'ETUDE ET L'EXPLOITATION DES PROCEDES GEORGES CLAUDE, 75007 Paris (FR); Institut national de recherche pour l'agriculture, l'alimentation et l'environnement, 75007 Paris (FR)
(72) Inventeur: Bertrandias, Aude, 78350 Les Loges-En-Josas (FR); Bremond, Ulysse, 78350 Les Loges-En-Josas (FR); Steyer, Jean-Philippe, 75007 PARIS (FR); Carrere, Hélène, 75007 PARIS (FR)
(74) Mandataire: Air Liquide

(57) **Abrégé**

Procédé de production de biogaz à partir d'un digestat issu d'un digesteur, ledit procédé comprenant :
a) Une étape de récupération du digestat en sortie d'un digesteur, d'un post-digesteur ou d'une cuve de stockage;
b) Une étape de séparation du digestat en un digestat solide et un digestat liquide ;
c) Une étape d'introduction du digestat solide dans au moins une cuve fermée ;
d) Une étape de digestion anaérobie dans la cuve sans chauffage ni brassage, et
e) Une étape de récupération du biogaz en sortie de la cuve.

## Description

La présente invention est relative à de production de biogaz à partir d'un digestat issu d'un digesteur.

Le biogaz est le gaz produit lors de la dégradation de matières organiques en l'absence d'oxygène (fermentation anaérobie) encore appelée méthanisation. Il peut s'agir d'une dégradation naturelle - on l'observe ainsi dans les marais ou les décharges d'ordures ménagères - mais la production de biogaz peut aussi résulter de la méthanisation de déchets dans un réacteur dédié, et dont les conditions sont contrôlées, appelé méthaniseur ou digesteur, puis éventuellement dans un post-digesteur similaire au digesteur et permettant de pousser plus loin la réaction de méthanisation.

On appellera biomasse tout groupement de matières organiques pouvant se transformer en énergie à travers ce processus de méthanisation e.g. boues de station d'épuration, fumiers/lisiers, résidus agricoles, cultures énergétiques, déchets alimentaires...En Europe, les résidus agricoles et cultures énergétiques (dédiées ou intermédiaires) représentent une grande part des biomasses traitées, notamment en Allemagne et Autriche, puis en France. En Allemagne, 15% des digesteurs agricoles sont opérés en mono-digestion, c'est-à-dire, avec 100% de cultures. La majorité des digesteurs mélangent les cultures avec du fumier/lisier (codigestion).

Le digestat est le résidu de la biomasse après digestion anaérobie. Le digestat est généralement pâteux, riche en carbone, azote, potassium, phosphore et oligoéléments. Ses propriétés pour la fertilisation et/ou amendement des sols sont reconnues, tout en étant d'origine biologique et générant moins d'odeurs que les lisiers ou fumiers classiquement épandus. Le digestat est épandu selon une procédure strictement encadrée.

Le digesteur, c'est-à-dire le réacteur dédié à la méthanisation de la biomasse, est une cuve fermée, chauffée ou non (opération à une température fixée, entre la température ambiante et 55°C) et dont le contenu constitué de la biomasse est brassé, en continu ou séquentiel. Les conditions dans le digesteur sont anaérobies et le biogaz généré se retrouve dans l'espace de tête du digesteur (ciel gazeux), où il est prélevé. Les post-digesteurs sont similaires aux digesteurs.

De par ses constituants principaux - méthane et dioxyde de carbone - le biogaz est un puissant gaz à effet de serre ; il constitue aussi, parallèlement, une source d'énergie renouvelable appréciable dans un contexte de raréfaction des énergies fossiles.

Le biogaz contient majoritairement du méthane (CH4) et du dioxyde de carbone (CO2) dans des proportions variables en fonction du mode d'obtention et du substrat mais peut également contenir, en moindres proportions de l'eau, de l'azote, de l'hydrogène sulfuré (H2S), de l'oxygène, ainsi que des composés organiques autres, à l'état de traces, dont le H2S, entre 10 et 50,000 ppmv.

Selon les matières organiques dégradées et les techniques utilisées, les proportions des composants diffèrent, mais en moyenne le biogaz comporte, sur gaz sec, de 30 à 75% de méthane, de 15 à 60% de CO2, de 0 à 15% d'azote, de 0 à 5% d'oxygène et des composés traces.

Le biogaz est valorisé de différentes manières. Il peut, après un traitement léger, être valorisé à proximité du site de production pour fournir de la chaleur, de l'électricité ou un mélange des deux (la cogénération).

Partant de là, un problème qui se pose est d'améliorer la production de biogaz.

Une solution de la présente invention est un procédé de production de biogaz à partir d'un digestat, ledit procédé comprenant :
a) Une étape de récupération du digestat en sortie d'un digesteur, d'un post-digesteur ou d'une cuve de stockage;
b) Une étape de séparation du digestat en un digestat solide et un digestat liquide ;
c) Une étape d'introduction du digestat solide dans au moins une cuve fermée ;
d) Une étape de digestion anaérobie dans la cuve sans chauffage ni brassage, et
e) Une étape de récupération du biogaz en sortie de la cuve.

Autrement dit la solution selon l'invention permet de récupérer le biogaz résiduel généré par le digestat solide.

Selon le cas, le procédé selon l'invention peut présenter une ou plusieurs des caractéristiques suivantes :
- l'étape de digestion anaérobie est effectuée avec inertage de la cuve avec du CO2 ou de l'azote,
- l'étape de digestion anaérobie présente une durée comprise entre 1 et 6 mois.
- le procédé est effectué pendant les mois d'été et l'étape de digestion présente une durée comprise entre 1 et 4 mois.
- le procédé est effectué pendant les mois d'hiver et l'étape de digestion présente une durée comprise entre 3 et 6 mois.
- la cuve est une cuve en béton fermée par une couverture rigide ou souple permettant l'étanchéité de la cuve après sa fermeture,
- les étapes c) et d) mettent en œuvre au moins deux cuves en parallèle,
- à chaque instant de l'étape d) on mesure la quantité de biogaz produite à partir du digestat solide, on compare cette quantité à une valeur cible et on arrête la digestion anaérobie du digestat solide lorsque la quantité de biogaz produit est supérieure à cette valeur cible. Ceci permet d'augmenter la quantité de biogaz généré tout en conservant les propriétés agronomiques du digestat solide (notamment teneur en carbone suffisante pour en faire un bon amendement).
- la valeur cible est comprise entre 25 et 400 Nm³ de biogaz par tonnes de matière volatile, de préférence entre 100 et 300 Nm³ de biogaz par tonnes de matière volatile.

La présente invention a également pour objet un procédé de production de biogaz à partir de biomasse, comprenant :
a) Une étape d'introduction de la biomasse dans un digesteur et/ou un post-digesteur,
b) Une étape de digestion anaérobie dans le digesteur et/ou le post-digesteur,
c) Une étape de production d'un premier flux de biogaz et de digestat,
d) Une étape de séparation du digestat en un digestat solide et un digestat liquide
e) Une étape d'introduction du digestat solide dans au moins une cuve fermée,
f) Une étape de digestion anaérobie dans la cuve sans chauffage ni brassage,
g) Une étape de récupération d'un deuxième flux de biogaz en sortie de la cuve, et
h) Une étape de mélange du premier flux de biogaz et du deuxième flux de biogaz de manière à former un flux M.

Le procédé de production de biogaz à partir de biomasse permet d'améliorer les rendements en biogaz des digesteurs car le biogaz résiduel est ainsi récupéré. Par ailleurs, cela améliore l'impact environnemental du digesteur, car il n'y a pas d'émissions de gaz à effet de serre dans l'atmosphère issu du digestat solide.

Selon le cas, le procédé de production de biogaz à partir de biomasse présente une ou plusieurs des caractéristiques ci-dessous :
- le flux M est épuré de manière à récupérer du biométhane et du CO2 et l'étape f) est réalisée avec inertage de la cuve avec le CO2 issu de l'épuration dudit flux mélangé.
- l'étape f) est réalisée avec inertage de la cuve à l'azote.
- Le procédé de production de biogaz à partir de biomasse comprend une étape d'introduction du digestat liquide dans une cuve de stockage, une étape de digestion anaérobie dans cette cuve de stockage et une étape de récupération d'un troisième flux de biogaz en sortie de cette cuve de stockage.
- le troisième flux de biogaz est mélangé au flux M.

Enfin le procédé de cogénération de chaleur et d'électricité à partir du flux M tel que défini à la revendication 10.

Dans le cadre de l'invention, le digestat solide est introduit dans une cuve de préférence en béton, de taille comprise entre 100 et 1000 m³. La cuve sera fermée par une couverture rigide ou souple permettant l'étanchéité de la cuve après sa fermeture. Aucun système de chauffage ni agitation n'est incorporé. Le chargement du digestat solide dans la cuve pourrait être réalisé manuellement par des tracteurs déjà présents sur site si la cuve est située à distance du séparateur du digestat en digestat liquide et digestat solide. Si par contre la cuve est à la base du séparateur de phase, le chargement s'opérera simplement par gravité. Puis, la digestion anaérobie se produira :
(i) soit lentement avec inertage naturel dans le temps,
(ii) soit avec inertage par du CO2, de préférence du CO2 issu de l'épuration du biogaz,
(iii) soit avec inertage à l'azote, de préférence de l'azote provenant d'un approvisionnement externe.

Pour la digestion anaérobie, le digestat solide sera laissé dans la cuve fermée de 1 à 6 mois : 1 à 4 mois pendant les mois d'été et 3 à 6 mois pendant les mois d'hiver car les cinétiques seront plus lentes à faible température. Notons que plusieurs cuves pourront être utilisées en parallèle pour traiter une plus grande fraction du digestat solide produit.

Après la digestion anaérobie, le deuxième flux de biogaz généré par le digestat solide sera récupéré en tête de cuve et mélangé au premier flux de biogaz généré par le digesteur. Ledit mélange sera ensuite épuré/cogénéré.

La cuve quant à elle sera vidée ensuite par exemple à l'aide d'un tracteur.

La solution selon l'invention permet donc, à moindres coûts, de récupérer le biogaz résiduel du digestat solide, sans impacter le fonctionnement du méthaniseur. Notamment, cette solution est intéressante quand le digesteur fonctionne au maximum de sa capacité, et donc que le digestat ne peut être recirculé. Aussi, cela permet d'améliorer le bilan carbone d'un site de méthanisation, en limitant les rejets de gaz à effet de serre et polluants dans l'atmosphère (dioxyde d'azote et dioxyde de carbone, ammoniac).

## Revendications

1. Procédé de production de biogaz à partir d'un digestat issu d'un digesteur, ledit procédé comprenant :
a) Une étape de récupération du digestat en sortie d'un digesteur, d'un post-digesteur ou d'une cuve de stockage;
b) Une étape de séparation du digestat en un digestat solide et un digestat liquide ;
c) Une étape d'introduction du digestat solide dans au moins une cuve fermée ;
d) Une étape de digestion anaérobie dans la cuve sans chauffage ni brassage, et
e) Une étape de récupération du biogaz en sortie de la cuve.

2. Procédé de production de biogaz selon la revendication 1, **caractérisé en ce que** l'étape de digestion anaérobie est effectuée avec inertage de la cuve avec du CO2 ou de l'azote.

3. Procédé de production selon l'une des revendications 1 ou 2, **caractérisé en ce que** l'étape de digestion anaérobie présente une durée comprise entre 1 et 6 mois.

4. Procédé de production de biogaz selon la revendication 3, **caractérisé en ce que** le procédé est effectué pendant les mois d'été et l'étape de digestion présente une durée comprise entre 1 et 4 mois.

5. Procédé de production de biogaz selon la revendication 3, **caractérisé en ce que** le procédé est effectué pendant les mois d'hiver et l'étape de digestion présente une durée comprise entre 3 et 6 mois.

6. Procédé de production de biogaz selon l'une des revendications 1 à 5, **caractérisé en ce que** la cuve est une cuve en béton fermée par une couverture rigide ou souple permettant l'étanchéité de la cuve après sa fermeture.

7. Procédé de production de biogaz selon l'une des revendications 1 à 6, **caractérisé en ce que** les étapes c) et d) mettent en œuvre au moins deux cuves en parallèle.

8. Procédé de production de biogaz selon l'une des revendications 1 à 7, **caractérisé en ce qu'**à chaque instant de l'étape d) on mesure la quantité de biogaz produite par le digestat, on compare cette quantité à une valeur cible et on arrête la digestion anaérobie du digestat solide lorsque la quantité de biogaz produite par le digestat est supérieure à cette valeur cible.

9. Procédé de production de biogaz selon la revendication 8, **caractérisé en ce que** la valeur cible est comprise entre 50 et 400 Nm³ de biogaz par tonnes de matière volatile, de préférence entre 100 et 300 Nm³ de biogaz par tonnes de matière volatile.

10. Procédé de production de biogaz à partir de biomasse, comprenant :
a) Une étape d'introduction de la biomasse dans un digesteur et/ou un post-digesteur,
b) Une étape de digestion anaérobie dans le digesteur et/ou un post-digesteur,
c) Une étape de production d'un premier flux de biogaz et de digestat,
d) Une étape de séparation du digestat en un digestat solide et un digestat liquide
e) Une étape d'introduction du digestat solide dans au moins une cuve fermée,
f) Une étape de digestion anaérobie dans la cuve sans chauffage ni brassage,
g) Une étape de récupération d'un deuxième flux de biogaz en sortie de la cuve, et
h) Une étape de mélange du premier flux de biogaz et du deuxième flux de biogaz de manière à former un flux M.

11. Procédé de production de biogaz selon la revendication 10, **caractérisé en ce que** le flux M est épuré de manière à récupérer du biométhane et du CO2 et l'étape f) est réalisée avec inertage de la cuve avec le CO2 issu de l'épuration dudit flux mélangé.

12. Procédé de production de biogaz selon la revendication 10, **caractérisé en ce que** l'étape f) est réalisée avec inertage de la cuve à l'azote.

13. Procédé de production de biogaz selon l'une des revendications 10 à 12, **caractérisé en ce qu'**il comprend une étape d'introduction du digestat liquide dans une cuve de stockage, une étape de digestion anaérobie dans cette cuve de stockage et une étape de récupération d'un troisième flux de biogaz en sortie de cette cuve de stockage.

14. Procédé de production de biogaz selon la revendication 13, **caractérisé en ce que** le troisième flux de biogaz est mélangé au flux M.

15. Procédé de cogénération de chaleur et d'électricité à partir du flux M tel que défini à la revendication 10.
